(19) Europäisches Patentamt
European Patent Office
Office européen des brevets

(11) Numéro de publication: **0 097 546**
**B1**

(12) **FASCICULE DE BREVET EUROPEEN**

(45) Date de publication du fascicule du brevet: **25.09.85**

(51) Int. Cl.[4]: **C 07 C 147/14**, A 61 K 31/135

(21) Numéro de dépôt: **83401048.0**

(22) Date de dépôt: **26.05.83**

(54) **Benzhydrylsulfinyléthylamines, procédé de préparation et utilisation en thérapeutique.**

(30) Priorité: **04.06.82 FR 8209804**

(43) Date de publication de la demande: **04.01.84 Bulletin 84/1**

(45) Mention de la délivrance du brevet: **25.09.85 Bulletin 85/39**

(84) Etats contractants désignés: **AT BE CH DE FR GB IT LI LU NL SE**

(56) Documents cités:
**FR - A - 2 326 181**

**J. ORG. CHEM., vol. 32, octobre 1967, pages 3191-3194, Washington, USA R.G. HISKEY et al.: "Chemistry of aliphatic disulfides. XIV. The preparation of disulfide sulfoxides by selective oxidation 1-3"**

**Le dossier contient des informations techniques présentées postérieurement au dépôt de la demande et ne figurant pas dans le présent fascicule.**

(73) Titulaire: **LABORATOIRE L. LAFON Société anonyme dite:, 1 rue Georges Médéric, F-94701 Maisons-Alfort (FR)**

(72) Inventeur: **Lafon, Louis, 5 rue de l'Alboni, F-75016 Paris (FR)**

(74) Mandataire: **Combe, André et al, CABINET BEAU DE LOMENIE 55 rue d'Amsterdam, F-75008 Paris (FR)**

EP 0 097 546 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention a trait à des benzhydrylsulfinyléthylamines en tant que produits industriels nouveaux. Elle concerne également l'utilisation de ces produits en thérapeutique ainsi que leur procédé de préparation.

On sait que le chlorhydrate de N-[2-(benzhydrylsulfinyl)éthyl]amine a été décrit en tant qu'intermédiaire de synthèse de dérivés disulfure-sulfoxydes par R.G. Hiskey et al., J. Org. Chem., *32*, pp. 3191-3194 (1967) sans étude de ses éventuelles propriétés thérapeutiques. On sait aussi que dans le brevet français N° 76.29137 et le brevet américain correspondant N° 4066686 on a proposé des dérivés du type N-(benzhydrylsulfinylalkyl)amines, notamment en tant que substances actives sur le système nerveux central (SNC). On vient de trouver de façon surprenante que de nouveaux dérivés de N-(benzhydrylsulfinylalkyl)-amine présentent des propriétés neuropsychopharmacologiques intéressantes par rapport aux produits objets des exemples 4 et 5 des brevets précités, à savoir les N-[2-(benzhydrylsulfinyl)-éthyl]morpholine et N-[2-(benzhydrylsulfinyl)-éthyl]pipéridine.

Les dérivés de N-benzhydrylsulfinylalkylamines selon l'invention sont caractérisés en ce qu'ils sont choisis parmi l'ensemble constitué par:

a) les N-[2-(benzhydrylsulfinyl)éthyl]amines de formule générale

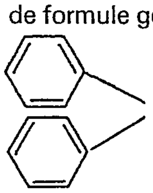
$CH-SO-CH_2-CH_2-NH-R$ (I)

(où R représente un groupe alkyle en $C_1$-$C_4$) et

b) leurs sels d'addition d'acide.

Parmi les groupes R inclus dans la définition donnée ci-dessus, on peut notamment mentionner les groupes $CH_3$, $C_2H_5$, i-$C_3H_7$ et t-$C_4H_9$.

Le composé le plus intéressant du point de vue psychopharmaceutique est le composé $R=CH_3$.

Parmi les sels d'addition d'acide qui conviennent, on peut notamment citer les sels d'addition non toxiques obtenus par réaction de la base libre de formule I avec un acide minéral ou organique. Parmi les acides qui conviennent à cet effet, on peut mentionner les acides chlorhydrique, bromhydrique, sulfurique, phosphorique, nitrique, picrique, formique, acétique, propionique, fumarique, maléique, malique, tartrique, citrique, oxalique, benzoïque, cinnamique, ascorbique, méthanesulfonique, paratoluènesulfonique, aspartique et glutamique.

Les produits de formule I sont actifs sur le SNC: ils agissent en tant que antidépresseurs du SNC, et présentent un effet antiagressif intéressant.

On préconise une composition thérapeutique qui est caractérisée en ce qu'elle renferme, en association avec un excipient physiologiquement acceptable, au moins un composé de formule I où l'un de ses sels d'addition non toxiques, en tant qu'ingrédient actif.

Les composés de formule I peuvent être préparés selon une méthode connue en soi par application de mécanismes réactionnels classiques. Le procédé que l'on préconise selon l'invention qui est illustré par le schéma:

$(C_6H_5)_2CH-SH$ (II)

+

$Br-CH_2CH_2-NHR$ (III)

$(C_6H_5)_2CH-S-CH_2CH_2-NHR$ (IV)

$H_2O_2$

$(C_6H_5)_2CH-SO-CH_2CH_2-NHR$ (I)

est caractérisé en ce que successivement

a) on fait réagir dans de l'eau en présence d'une base notamment choisie parmi NaOH et KOH, le diphénylméthanethiol (II) avec une 2-bromoéthylamine de formule $Br-CH_2CH_2-NHR$ (III) — où R est défini comme ci-dessus — au reflux, pendant au moins 1 h, à raison de 1 à 1,2 mol de III pour 1 mol de II, pour obtenir une benzhydrylthioéthylamine de formule:

$(C_6H_5)_2CH-S-CH_2CH_2-NHR$ (IV)

puis,

b) on soumet la benzhydrylthioéthylamine ainsi obtenue à une réaction d'oxydation dans l'acide acétique au moyen de $H_2O_2$ à 110-130 volumes, à une température de 40-45° C pendant 1 h.

Un certain nombre de N-[2-(benzhydrylsulfinyl)éthyl]amines selon l'invention a été consigné de façon non limitative dans le tableau I ci-après:

*Tableau I*

$(C_6H_5)_2CH-SO-CH_2CH_2-NHR$

| Produit | N° de code | R | Point de fusion |
|---|---|---|---|
| Ex. 1 (a) | CRL 40883 | $CH_3$ | 128-130° C |
| Ex. 2 (a) | — | $CH(CH_3)_2$ | — |
| Ex. 3 (a) | — | $C(CH_3)_3$ | — |
| *Note:* (a) = chlorhydrate | | | |

D'autres avantages et caractéristiques de l'invention seront mieux compris à la lecture qui va suivre d'exemples de préparation nullement limitatifs.

*Préparation I:*

*Obtention du chlorhydrate de N-[2-(benzhydrylsulfinyl)éthyl]méthylamine.*

(Exemple 1; N° de code: CRL 40883.)

*1. Chlorhydrate de N-[2-(benzhydrylthio)-éthyl]méthylamine.*

15 g (0,075 mol) de diphénylméthanethiol en suspension dans 75 ml d'eau sont salifiés avec

16 g (0,4 mol) de soude en pastilles en solution dans 25 ml d'eau. On porte le mélange à reflux et coule goutte à goutte dans ce mélange, une solution de 17,6 g (0,08 mol) de bromhydrate de N-(2-bromoéthyl)méthylamine dans 50 ml d'eau. On maintient une heure à reflux, refroidit, extrait à l'éther, lave à l'eau. On extrait la solution éthérée avec 100 ml d'acide HCl 2N, on précipite la base avec NaOH concentrée. La base est reprise à l'éther, on lave la phase éthérée à l'eau, sèche et précipite le chlorhydrate désiré par addition d'éthanol chlorhydrique. On essore le précipité, le lave à l'acétate d'éthyle et par recristallisation dans l'éthanol, on obtient le chlorhydrate de N-[2-(benzydrylthio)éthyl]méthylamine avec un rendement de 71%. F=121-122°C.

*2. CRL 40883:*

14 g (0,05 mol) de chlorhydrate de N-[2-(benzhydrylthio)éthyl])méthylamine en solution dans 50 ml d'acide acétique sont oxydés au moyen de 5 ml de $H_2O_2$ à 110 volumes pendant 1 h à 40°C. On évapore l'acide acétique sous vide, reprend le résidu d'évaporation à l'acétone et essore le précipité formé. Par recristallisation dans le mélange éthanol-acétate d'éthyle (1:1) v/v, on obtient le CRL 40883 avec un rendement de 58%. F=128-130°C.

On a résumé ci-après les résultats des essais neuropsychopharmacologiques qui ont été entrepris avec le CRL 40883 (produit de l'exemple 1). Dans ces essais le CRL 40883 en solution dans de l'eau distillée à pH 5 a été administré par voie intrapéritonéale sous un volume de 20 ml/kg chez la souris mâle et sous un volume de 5 ml/kg chez le rat mâle.

*A) Toxicité:*

Chez la souris mâle la DL-30 par voie I.P. du CRL 40883 est de l'ordre de 250 mg/kg.

*B) Comportement global et réactivités:*

Des lots de 6 animaux sont observés avant, puis 15 min, 30 min, 1 h, 2 h, 3 h et 24 h après l'administration de CRL 40883.

1) Chez la souris:

Aux doses de 64 mg/kg, 16 mg/kg, 4 mg/kg et 1 mg/kg, le CRL 40883 n'entraîne pas de modifications sensibles du comportement et des réactivités. On observe une sédation modérée surtout à la dose de 128 mg/kg.

2) Chez le rat:

A la dose de 32 mg/kg, on observe une hyporéactivité au toucher et une hypotonie musculaire pendant 30 min; aux doses de 8 mg/kg, 2 mg/kg et 0,5 mg/kg on n'observe pas de symptômes particuliers.

*C) Action sur le SNC:*

Le CRL 40883 présente les effets d'une substance antidépressive (antagonisme des hypothermies induites par l'apomorphine, la réserpine ou l'oxotrémorine) ainsi qu'une potentialisation des stéréotypies amphétaminiques.

Par ailleurs aux doses de 16 mg/kg et de 64 mg/kg, le CRL 40883 entraîne une forte diminution du nombre de combats dans les groupes de souris, selon le test d'agressivité intergroupes.

## Revendications

1. Dérivés de N-(benzhydrylsulfinylalkyl)-amine, caractérisés en ce qu'ils sont choisis parmi l'ensemble constitué par:

a) les N-[2-(benzhydrylsulfiñyl)éthyl]amine de formule générale

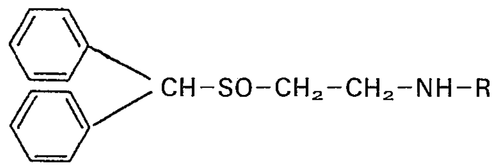

(I)

(où R représente un groupe alkyle en $C_1$-$C_4$), et

b) leurs sels d'addition d'acide.

2. Dérivés selon la revendication 1, caractérisés en ce que dans la formule I le groupe R représente $CH_3$, $CH_2$,$CH_3$, i-$C_3H_7$ ou t-$C_4H_9$.

3. N-[2-(benzhydrylsulfinyl)éthyl]méthylamine et ses sels d'addition d'acide.

4. Composition thérapeutique, caractérisée en ce qu'elle renferme, en association avec un excipient physiologiquement acceptable au moins un dérivé de N-[2-(benzhydrylsulfinyl)éthyl]amine selon l'une quelconque des revendications 1 à 3 ou l'un de ses sels d'addition d'acide non toxiques.

5. Procédé de préparation d'un dérivé de formule I selon la revendication 1, caractérisé en ce que successivement:

a) on fait réagir dans de l'eau en présence d'une base notamment choisie parmi NaOH et KOH, le diphénylméthanethiol de formule

$$(C_6H_5)_2CH-SH \qquad (II)$$

avec une 2-bromoéthylamine de formule

$$Br–CH_2CH_2–NHR \qquad (III)$$

(où R est défini comme ci-dessus), au reflux pendant au moins 1 h, à raison de 1 à 1,2 mol de III pour 1 mol de II, pour obtenir une benzhydrylthioéthylamine de formule:

$$(C_6H_5)_2CH-S-CH_2CH_2-NHR \qquad (IV)$$

puis,

b) on soumet la benzhydrylthioéthylamine ainsi obtenue à une réaction d'oxydation dans l'acide acétique au moyen de $H_2O_2$ à 110-130 volumes, à une température de 40-45°C pendant 1 h.

## Patentansprüche

1. N-(Benzhydrylsuflinylalkyl)amin-Derivate, dadurch gekennzeichnet, dass sie ausgewählt sind aus der Gruppe bestehend aus

a) den N-[2-(Benzhydrylsulfinyl)äthyl]aminen der allgemeinen Formel

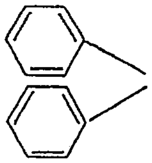$CH-SO-CH_2-CH_2-NH-R$ (I)

(worin R eine $C_1$-$C_4$-Alkylgruppe darstellt) und

b) ihren Säureadditionssalzen.

2. Derivate nach Anspruch 1, dadurch gekennzeichnet, dass die Gruppe R in der Formel I $CH_3$, $CH_2CH_3$, i-$C_3H_7$ oder t-$C_4H_9$ bedeutet.

3. N-[2-(Benzhydrylsulfinyl)äthyl]methylamin und seine Säureadditionssalze.

4. Therapeutische Zusammensetzung, dadurch gekennzeichnet, dass sie in Verbindung mit einem physiologisch akzeptablen Exzipienten mindestens ein N-[2-(Benzhydrylsulfinyl)äthyl]-amin-Derivat nach einem der Ansprüche 1 bis 3 oder eines seiner nichttoxischen Säureadditionssalze enthält.

5. Verfahren zur Herstellung eines Derivats der Formel I nach Anspruch 1, dadurch gekennzeichnet, dass man nacheinander:

a) in Wasser in Gegenwart einer Base, insbesondere ausgewählt aus NaOH und KOH, Diphenylmethanthiol der Formel

$$(C_6H_5)_2CH-SH \quad (II)$$

mit einem 2-Bromäthylamin der Formel

$$Br-CH_2CH_2-NHR \quad (III)$$

(worin R obige Bedeutung hat) unter Rückfluss während mindestens 1 h in einer Menge von 1 bis 1,2 Mol von III pro 1 Mol von II zur Gewinnung eines Benzhydrylthioäthylamins der Formel

$$(C_6H_5)_2CH-S-CH_2CH_2-NHR \quad (IV)$$

zur Umsetzung bringt und

b) das so erhaltene Benzhydrylthioäthylamin einer Oxidationsreaktion in Essigsäure mittels $H_2O_2$ mit 110 bis 130 Volumenteilen bei einer Temperatur von 40 bis 45°C während 1 h unterzieht.

## Claims

1. Derivatives of N-(benzhydrylsulfinylalkyl)-amine, characterized in that they are selected from among the group constituted by:

a) the N-[2-(benzhydrylsulfinyl)ethyl]amines of general formula

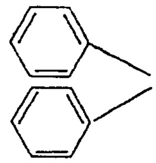$CH-SO-CH_2-CH_2-NH-R$ (I)

(wherein R represents a $C_1$-$C_4$ alkyl group), and

b) their acid addition salts.

2. Derivatives according to claim 1, characterized in that in formula I the group R represents $CH_3$, $CH_2CH_3$, i-$C_3H_7$ or t-$C_4H_9$.

3. N-[2-(benzhydrylsulfinyl)ethyl]methylamine and its acid addition salts.

4. Therapeutical composition, characterized in that it contains, in association with a physiologically acceptable excipient, at least one derivative of N-[2-(benzhydrylsulfinylethyl]amine according to anyone of claims 1 to 3 or one of its non toxic acid addition salts.

5. Preparation process of a derivative of formula I according to claim 1, characterized in that it successively comprises the steps of:

a) reacting in water in the presence of a base particularly selected from among NaOH and KOH, the diphenylmethanethiol of formula

$$(C_6H_5)_2\ CH-SH \quad (II)$$

with a 2-bromoethylamine of formula

$$Br-CH_2CH_2-NHR \quad (III)$$

(wherein R is defined as above), at reflux for at least one hour, at the rate of 1 to 1.2 mole of III for one mole of II, to obtain a benzhydrylthioethylamine of formula:

$$(C_6H_5)_2CH-S-CH_2CH_2-NHR \quad (IV)$$

then,

b) subjecting the benzhydrylthioethylamine thus obtained at an oxydation reaction in acetic acid by means of $H_2O_2$ at 110-130 volumes, at a temperature of 40-45°C for one hour.